# EUROPÄISCHE PATENTSCHRIFT

(11) **EP 1 731 121 B1**
(45) Veröffentlichungstag und Bekanntmachung des Hinweises auf die Patenterteilung: **14.07.2010**
(21) Anmeldenummer: 06009621.1
(22) Anmeldetag: 10.05.2006
(51) Int. Cl.: A61F 11/08

(54) **Gehörschutzstöpsel**
Ear plug
Bouchon protection auriculaire

(30) Priorität: 10.06.2005 DE 202005009132 U
(43) Veröffentlichungstag der Anmeldung: 13.12.2006
(73) Patentinhaber: Uvex Arbeitsschutz GmbH, 90766 Fürth (DE)
(72) Erfinder: Brück, Stefan, 90419 Nürnberg (DE)
(74) Vertreter: Schneck, Herbert

(56) Entgegenhaltungen:
- WO-A-2005/023147
- FR-A- 1 340 726
- US-A1- 5 044 463

## Beschreibung

Die Erfindung richtet sich auf einen Gehörschutzstöpsel zum Einführen in den Gehörgang aus einem weichen Material, insbesondere Schaumstoff, mit einer zur Außenseite hin offenen Ausnehmung.

Ein derartiger Gehörschutzstöpsel ist aus US 5,044,463 bekannt. Die vorbekannte Längsausnehmung soll dazu dienen, den Tragekomfort zu erhöhen.

Aus WO 02/43633 A1 ist ein Gehörschutzstöpsel bekannt, der mit einem Griff aus härterem, formstabilerem Material versehen ist, wobei am Griffende Vertiefungen zur Anbringung einer Kordel vorgesehen sind.

Auch WO 2005/023 147 A2 beschreibt einen Gehörschutzstöpsel mit einem weichen Schaumstoffgrundkörper und einem härteren Kern, der das Einführen erleichtern soll. Eine X-förmige Aussparung am Vorderende dient zum Entfernen des harten Kernendes zur Erhöhung des Tragekomforts.

Hiervon ausgehend liegt der Erfindung die Aufgabe zugrunde, einen gattungsgemäß bekannten Gehörschutzstöpsel so zu verbessern, dass nicht nur ein noch weiterer erhöhter Tragekomfort, sondern insbesondere auch eine einfache Handhabung beim Einsetzen und Herausnehmen in das Ohr bzw. aus dem Ohr erreicht werden.

Diese Aufgabe wird erfindungsgemäß dadurch gelöst, dass die Längsausnehmung im Querschnitt gesehen eine annähernd kreuzförmige Konfiguration aufweist, und dass der Gehörschutzstöpsel aus einem Schaumstoff besteht.

Durch die erfindungsgemäße Konfiguration der Ausnehmung wird erreicht, dass an der flachen Stöpseloberseite bzw. ―außenseite mit den dort vorhandenen relativ harten Kanten eine höhere Weichheit und Nachgiebigkeit erreicht wird, die den Tragekomfort gegenüber einer zylindrischen Längsausnehmung nochmals erhöht, weil der Rand insgesamt geschmeidiger wird.

Durch die kreuzförmige Konfiguration lässt sich der Stöpsel beim Einführen optimal Zusammendrücken, so dass der Einführvorgang erleichtert wird. Zum Herausnehmen kann der Stöpsel mit den Fingernägeln im Bereich der Ausnehmung erfasst und zuverlässig gehalten werden.

Eine sich vom Boden wegerstreckende zusätzliche Längsausnehmung ermöglicht eine gezielte Einstellung der Dämpfungseigenschaften z.B. so, dass eine Rest-Hörfähigkeit trotz Dämpfung des allgemeinen Pegels erhalten bleibt.

Nachfolgend wird die Erfindung anhand eines bevorzugten Ausführungsbeispiels in Verbindung mit der Zeichnung näher erläutert. Dabei zeigen:
- Fig. 1: eine perspektivische Ansicht eines erfindungsgemäßen Gehörschutzstöpsels von schräg oben.
- Fig. 2: eine Seitenansicht,
- Fig. 3: eine Aufsicht auf die äußere Stirnseite und
- Fig. 4: einen Schnitt längs der Linie A-A in Fig. 3.

Ein in der Zeichnung dargestellter Gehörschutzstöpsel 1 besteht aus einem sich langsam erholenden Polyurethanschaum und ist durch Gießen hergestellt.

Der Gehörschutzstöpsel 1 umfasst einen sich zur Innenseite hin konisch verjüngenden Grundkörper 2, ein abgerundetes inneres Ende 3 und ein flaches äußeres Ende 4.

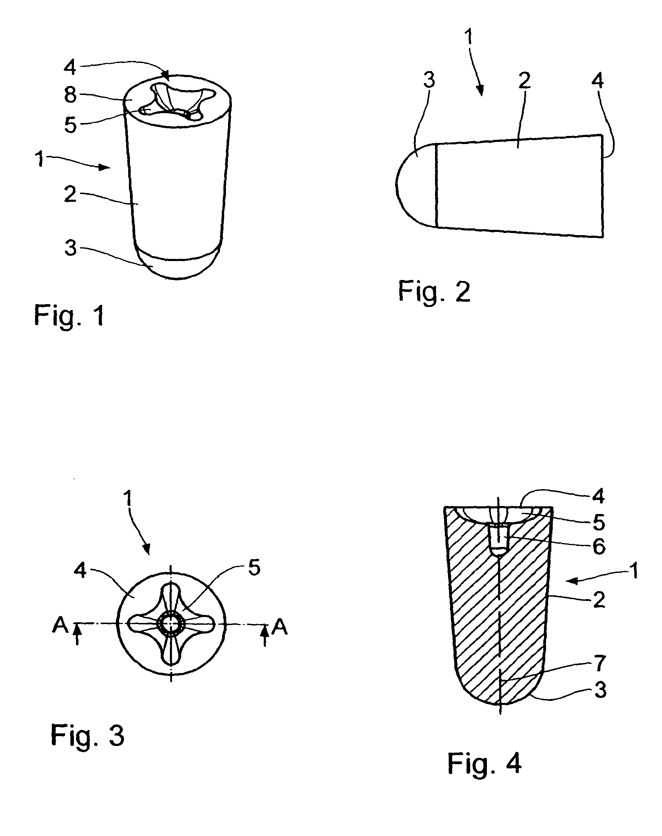

An dem flachen äußeren Ende 4 ist eine Ausnehmung 5 ausgebildet, welche eine kreuzförmige Grundform aufweist mit, wie in Fig. 3 erkennbar, abgerundeten Übergängen.

An die verhältnismäßig flache Ausnehmung 5 schließt sich eine Längsausnehmung 6 an, welche längs der Längsachse 7 des Grundkörpers 2 verläuft.

Die kreuzförmige Ausnehmung 5 dient dazu, den Randbereich 8 der Stirnseite am Ende 4 geschmeidiger zu machen und hierzu den Tragekomfort zu erhöhen. Gleichzeitig lässt sich das Ende 4 beim Einführen des Stöpsels aufgrund der Konfiguration der Ausnehmung 5 gut Zusammendrücken, wodurch das Einführen erleichtert wird und andererseits nach dem Einführen eine gleichmäßige, definierte Anlage im Ohr gewährleistet ist. Die Ausnehmung 5 ist auch beim Herausnehmen des Gehörschutzstöpsels 1 hilfreich, weil hierdurch das Ergreifen des Stöpsels wesentlich erleichtert wird.

## Patentansprüche

1. Gehörschutzstöpsel zum Einführen in den Gehörgang aus einem weichen Material mit einer zur Außenseite hin offenen Ausnehmung, **dadurch gekennzeichnet, dass** die Ausnehmung (5) im Querschnitt gesehen eine annähernd kreuzförmige Konfiguration aufweist, und dass der Gehörschutzstöpsel aus einem Schaumstoff besteht.

2. Gehörschutzstöpsel nach Anspruch 1, **dadurch gekennzeichnet, dass** die Außenkontur der Ausnehmung (5) im Querschnitt abgerundet ausgebildet ist.

3. Gehörschutzstöpsel nach Anspruch 1, **dadurch gekennzeichnet, dass** sich vom Boden der relativ flachen kreuzförmigen Ausnehmung (5) in axialer Richtung eine Längsausnehmung (6) nach innen erstreckt.

## Claims

1. A hearing protection earplug for insertion into the ear canal ear composed of a soft material having a cavity that is open toward the outside, **characterized in that** the cavity (5), as seen in the cross-section, has an approximately cross-shaped configuration, and that the hearing protection earplug is made of a foam material.

2. A hearing protection earplug according to claim 1, **characterized in that** the outer contour of the cavity (5) is designed rounded in cross-section.

3. A hearing protection earplug according to claim 1, **characterized in that** a longitudinal cavity (6) extends inward in the axial direction from the base of the relatively shallow cross-shaped cavity (5).

## Revendications

1. Bouchon de protection auditive destiné à être introduit dans le conduit auditif, en un matériau mou avec un évidement ouvert vers le côté extérieur, **caractérisé en ce que** l'évidement (5) présente, vu en section transversale, une configuration approximativement en forme de croix et **en ce que** le bouchon de protection auditive se compose de mousse synthétique.

2. Bouchon de protection auditive selon la revendication 1, **caractérisé en ce que** le contour extérieur de l'évidement (5) est réalisé de manière arrondie en section transversale.

3. Bouchon de protection auditive selon la revendication 1, **caractérisé en ce qu'**un évidement longitudinal (6) s'étend vers l'intérieur du fond de l'évidement (5) en forme de croix relativement plat dans le sens axial.
